# EUROPEAN PATENT APPLICATION

(11) **EP 4 744 610 A1**
(43) Date of publication of application: **20.05.2026**
(21) Application number: 24213018.5
(22) Date of filing: 14.11.2024
(51) Int. Cl.: A61B 34/20, G16H 20/40, A61B 34/10, A61B 90/00

(54) **TECHNIQUE FOR SUPPORTING A SURGEON IN VALIDATING AN IMPLANT PLACEMENT**

(71) Applicant: Stryker European Operations Limited, Carrigtwohill, Co. Cork T45 HX08 (IE)
(72) Inventor: SINGH BEDI, Inderjeet, 110032 Delhi (IN); WEEDE, Oliver, 79110 Freiburg (DE); RAINA, Ravi Kiran, 201301 Noida (IN); KHORWAL, Renu, 110063 New Delhi (IN); KUMAR, Pankaj, 110005 New Delh (IN)
(74) Representative: Schott, Jakob Valentin

(57) **Abstract**

A computer-implemented method (1000) for supporting a surgeon in validating an implant placement, the method (1000) comprising: obtaining (S1010) preoperative patient image data comprising representations of a plurality of anatomical elements of a patient's body; obtaining (S1020) implant placement data indicative of a pose of at least one placed implant relative to at least one of the anatomical elements; obtaining (S1030) tracking data indicative of a change of a relative pose of at least one of the anatomical elements; determining (S1040), based on the preoperative patient image data, the implant placement data and the tracking data, a first simulated patient image comprising representations of the plurality of anatomical elements with the at least one of the anatomical elements having the changed pose, the simulated patient image further comprising a visualization of the placed implant; and triggering (S1050) display of the first simulated patient image to support the surgeon in validating the placement of the implant.

## Description

### Technical Field

The present disclosure generally relates to a computer-implemented method for supporting a surgeon in validating an implant placement. A surgical navigation system and a computer program are also provided.

### Background

Present surgical navigation systems are configured to generate a 2D image based on a 3D image dataset of a patient anatomy, at any selected plane within the volumetric data, with a particular emphasis on depicting lateral (LAT) and anterior-posterior (AP) views, as these views are frequently utilized in spinal surgery. The generated 2D image might be a Digitally Reconstructed Radiograph (DRR) or a 2D slice generated from a 3D image data set, also referred to as Slice Reconstruction.

In present systems, validation steps (e.g., at the end of spinal surgery or surgical phases) are notably absent. For example, such systems implement the following workflow steps:
1. Planning: Surgeon reviews and pre-plans the surgical case, including implant placement
2. Setup and Registration: Instruments setup and patient registration is done.
3. Navigation: Surgeon navigates the instruments and places the implants to perform the surgical procedure.

Once an implant has been placed, it is common for the surgeon to acquire an X-ray image of the patient's body and the placed implant using an imaging device such as a C-arm, and to review the acquired image for implant placement validation.

There is a need for supporting a surgeon in validating placement of an implant.

### Summary

The present disclosure provides a technique that solves the above or other problems.

According to a first aspect, a computer-implemented method for supporting a surgeon in validating an implant placement is provided. The method comprises: obtaining preoperative patient image data comprising representations of a plurality of anatomical elements of a patient's body; obtaining implant placement data indicative of a pose of at least one placed implant relative to at least one of the anatomical elements; obtaining tracking data indicative of a change of a relative pose of at least one of the anatomical elements; determining, based on the preoperative patient image data, the implant placement data and the tracking data, a first simulated patient image comprising representations of the plurality of anatomical elements with the at least one of the anatomical elements having the changed pose, the simulated patient image further comprising a visualization of the placed implant; and triggering display of the first simulated patient image to support the surgeon in validating the placement of the implant.

The preoperative patient image data may be obtained from an external source comprising means for capturing said data. In particular, the external source may be, for example, a medical imaging unit such as an X-ray scanner or a computer tomography (CT) scanner. The preoperative patient data may be or comprise magnetic resonance (MR) image data and/or CT image data.

The implant placement data may be predetermined. This placement data may be input by the surgeon or another medical professional based on the surgical scenario and/or the pose of the anatomical elements of the patient's body (e.g. as represented by the preoperative patient image data). Additionally, or alternatively, the implant placement data may be generated and/or obtained after the implant has been placed. The implant placement data may be derived from a medical image depicting at least a portion of the placed implant and at least a part of the patient's body (e.g., a medical image of intraoperative or postoperative patient image data).

The tracking data may be obtained from the same external source as the preoperative patient image data and the implant placement data, or a different external source. The tracking data may be obtained from a tracking system, for example an optical tracking system. The tracking data may be indicative of a change of a pose of an anatomy tracker relative to another anatomy tracker or relative to a global patient tracker. Such an anatomy tracker may be attached to a bone of the patient's body, for example a vertebra.

The first simulated patient image may be a simulated medical image. The first simulated patient image may be displayed to the surgeon using any suitable display means such as, for example, a screen, a monitor, or any other means.

In some examples, the preoperative patient image data comprises 3D image data and the first simulated patient image is a 2D image or a 3D image. In some examples, a 3D image data set for navigation is used for the method, wherein the data comes from, for example, an X-ray scanner or a CT scanner. Based at least partly on this data, the first simulated image can be (automatically) generated, wherein this image can be 2D or 3D, depending on the scenario the method is used in. This may allow for a better evaluation of the placement of the implant, and also improved validation of said placement.

In some examples, the tracking data is obtained via at least one (e.g., anatomy) tracker attached to at least one of the anatomical elements, and wherein the first simulated patient image is determined at least partly based on the tracking data obtained via the at least one tracker. The capturing of the position of the at least one of the anatomical elements may be achieved by attaching (e.g., anatomy) trackers to the anatomical element(s) and subsequently updating a model containing the segmented anatomical element(s). This may provide for an improved evaluation and comparison of the placement of the implant.

In some examples, the first simulated patient image is determined at least partly based on a computer-implemented deformable registration method, wherein an input of the computer-implemented deformable registration method is the obtained tracking data. A deformable registration method, such as, for example, Elastix, may be employed. Here, only the bony structures of the anatomical element(s) may be treated as rigid during the registration process. This may provide for an improved evaluation and comparison of the placement of the implant and/or the preoperative patient data.

In some examples, the implant placement data is determined based on tracking data indicative of a pose of the at least one placed implant and tracking data indicative of a simultaneous pose of the at least one of the anatomical elements. This may allow for an improved comparison of the positioning of the implant with a planned positioning of the implant, and an improved implant placement validation process.

In some examples, the method further comprises adding a mesh of one or more of the at least one implant to the preoperative patient image data; and intersecting the mesh with an image plane of the first simulate patient image that is to be determined, wherein an indication of an implant contour resulting from the intersecting is visualized in the first simulated patient image. The mesh of the planned implant may be added to the 3D preoperative image data set. An appropriate plane for this mesh and/or data may be chosen by the surgeon. Such planes may, for example, include the implant trajectory such as "Implant Axial" and "Implant Sagittal" planes. The mesh of the implant may be intersected with the plane, and the resulting contour may be visualized in the first simulated patient image. This may improve the validation of the placement of the implant.

In some examples, the first simulated patient image is constructed with respect to a plane parallel with a longitudinal axis of one or more of the at least one placed implant, or a plane perpendicular to the longitudinal axis of one or more of the at least one placed implant. This may improve the validation of the placement of the implant.

In some examples, the first simulated patient image is determined at least partly based on a computer-implemented ray casting algorithm configured to generate a Hounsfield unit value for at least part of the at least one of the anatomical elements, and wherein the generated Hounsfield unit value is configured to be used to generate a Digitally Reconstructed Radiograph, DRR, for the first simulated patient image. An algorithm for generating the DRR may be, for example, the Seddon and Jacobs algorithm, which creates digitally reconstructed radiographs out of 3D image data sets. It is a ray casting algorithm with the ray source at the isocenter of a simulated C-Arm. All Hounsfield unit values, or a subset thereof, on a single ray may be integrated (summed up) to render a pixel on the DRR. The process may be repeated for each of the pixels of the DRR, or a subset thereof. This may improve the comparison and validation of the placement of the implant.

In some examples, before or after triggering the display of the first simulated patient image, altering the first simulated patient image based on the generated Hounsfield unit value, in particular wherein the first simulated patient image is altered based on one or more of: a first Hounsfield unit value range (e.g., below 100) corresponding to soft tissue; a second Hounsfield unit value range (e.g., between 250 and 2.000) corresponding to bone; and a third Hounsfield unit value range (e.g., above 3.000) corresponding to the at least one placed implant. To visualize bony structures and/or soft tissues, the Hounsfield unit values can be filtered automatically and/or by the surgeon. It may be possible to differentiate between soft tissues and bony structure based on the Hounsfield value ranges: Soft tissues typically have Hounsfield unit values ranging from about 0 to about 100 HU on a typical CT scan. However, the exact Hounsfield unit values can vary depending on factors such as, for example, the specific soft tissue being imaged. Bones typically have higher Hounsfield unit (HU) values compared to soft tissues due to their greater density. The exact HU values for bones can vary depending on factors such as the type of bone and its composition. However, the HU values for bones typically range from about 700 to about 3000 HU. A different (e.g., higher) range may be used for the implant. The surgeon may be provided with an option to render/filter out any of the above by selecting appropriate ranges of Hounsfield unit values. This may improve the comparison and validation of the placement of the implant.

In some examples, the implant placement data further comprises a volume of the at least one placed implant, and wherein a virtual object with a Hounsfield unit value matching a material of the implant is placed into the preoperative patient image data. To include implants, such as, for example, screws in the generated image, the implant attributes (position, geometry, material) may be considered. The respective volume of the implants in the image data set may be calculated. An object with the appropriate Hounsfield unit values matching the implant material may be placed in the 3D preoperative image data set. The same can be applied to other implants such as, for example, cages. After placing the object, the DRR may be computed. This may improve the comparison and validation of the placement of the implant.

In some examples, the simulated first patient image is triggered to be displayed together with at least one of: a pre-operative medical patient image such as a fluoroscopic image; an initial simulated patient image; an indication of a result of an intermediate step of the method; an indication of a planned implant; and a second simulated patient image differing from the first simulated patient image (e.g., in poses of one or more anatomical elements and/or implants). Images such as, for example, imported fluoro shots or simulated 2D Spine Images, at the end of the surgery or a surgical phase, may be displayed in parallel or superimposed to a pre-operative patient image (e.g., fluoro shot) and/or the initial generated 2D spine image. Such a display may be conducted:
a. During any previous intermediate step;
b. After changing the anatomy of the patient, for example by performing disc spreading, bone resection;
c. After placement of an implant; or
d. Once all the implants are placed and procedure is finished.

The above may lead to an improved comparison and validation of the placement of the placed implant with respect to the planned placement of the implant.

In some examples, the method further comprises determining a difference between a pose of at least one planned implant and a pose of the at least one placed implant, and triggering display of an indication of the difference. This may improve the comparison and validation of the placement of the implant.

In some examples, the method further comprises determining a deviation between a pose of an anatomical element as represented by the patient image and a changed pose of the anatomical element as indicated by the tracking data, and triggering display of an indication of the deviation. This may improve the comparison and validation of the placement of the implant.

In some examples, the method further comprises obtaining user input, wherein the first simulated patient image is triggered to be displayed in response to the obtained user input. The surgeon can compare the complete spine structure or select a customized area for comparison. The surgeon may be able to select a previous state from the image history. This may improve the comparison and validation of the placement of the implant.

In some examples, an axis and/or a linear border of the at least one of the anatomical elements, as represented in the first simulated patient image, is triggered to be displayed in response to the obtained user input. The surgeon can select the display mode and/or the perspective of the image such as, for example, an axial, sagittal or coronal plane, an alignment to a specific fluoro shot, or an alignment to a specific planned or placed implant. This may improve the comparison and validation of the placement of the implant.

In some examples, an indication of at least one spinal parameter derived from the first simulated patient image is triggered to be displayed together with the first simulated patient image. The display of this parameter may be used as a basis for generating a final patient report and the associated documentation in a quicker and more efficient manner.

In some examples, the at least one spinal parameter comprises one or more of:
- An indication of a Gertzbein-Robbins grading scale;
- A lordotic angle and/or a kyphotic angle and/or a Cobb angle between different ones of the plurality of anatomical elements, in particular between fused vertebrae;
- A curve visualizing a curvature of the plurality of anatomical elements, in particular a spinal curvature of immobilized vertebrae;
- Placement data indicative of a pose of at least one further placed implant relative to at least one of the anatomical elements, in particular a rod; and
- A deviation between the pose of at least one planned implant and the pose of the at least one placed implant, in particular wherein the implant is a screw.

The display of any one of the above-mentioned parameters may be used as a basis for generating a final patient report and the associated documentation in a quicker and more efficient manner, and allow the surgeon to more easily validate the placement of the implant and/or compare the planned placement of the implant to the actual placement of the implant.

According to a second aspect, we describe a surgical navigation system comprising at least one processor configured to carry out the method of the first aspect.

In some examples, the system further comprises at least one of the following entities: a tracking system configured to generate the tracking data; at least one tracker trackable by the tracking system and attachable to at least one anatomical element of the patient; a display unit configured to display at least the first simulated patient image; a user input unit configured to receive user input. The use, and types, of such systems, trackers and unit is known to the skilled person.

According to a third aspect, we describe a computer program comprising instructions which, when the program is executed by at least one processor, cause the at least one processor to carry out the method of the first or second aspect, wherein the program is optionally carried by a carrier such as a data signal, a portable memory device or a non-transitory computer storage medium.

In some examples, not all of the steps mentioned in relation to the first aspect are required. In some examples, the steps may be in a different order. In some examples, some of the steps happen simultaneously.

It is clear to a person skilled in the art that the statements set forth herein may be implemented under use of hardware circuits, software means, or a combination thereof. The software means can be related to programmed microprocessors or a general computer, an ASIC (Application Specific Integrated Circuit) and/or DSPs (Digital Signal Processors). For example, the processing unit may be implemented at least partially as a computer, a logical circuit, an FPGA (Field Programmable Gate Array), a processor (for example, a microprocessor, microcontroller (µC) or an array processor)/a core/a CPU (Central Processing Unit), an FPU (Floating Point Unit), NPU (Numeric Processing Unit), an ALU (Arithmetic Logical Unit), a Coprocessor (further microprocessor for supporting a main processor (CPU)), a GPGPU (General Purpose Computation on Graphics Processing Unit), a multi-core processor (for parallel computing, such as simultaneously performing arithmetic operations on multiple main processor(s) and/or graphical processor(s)) or a DSP.

It is further clear to the person skilled in the art that even if the herein-described details are described in terms of a method, these details may also be implemented or realized in a suitable device, apparatus, system, computer processor, or memory connected to a processor, wherein the memory can be provided with one or more programs that perform the method, when executed by the processor. Therefore, methods like swapping and paging can be deployed.

Even if some of the aspects described above have been described in reference to the method, these aspects may also apply to the system and computer program and vice versa.

### Brief Description of the Drawings

These and other aspects of the invention will now be further described, by way of example only, with reference to the accompanying figures, wherein like reference numerals refer to like parts, and in which:
- Figure 1: shows a screenshot of a known method of supporting a surgeon during implant placement;
- Figure 2: shows a flowchart of supporting a surgeon in validating an implant placement according to some examples as described herein;
- Figure 3: shows a screenshot of supporting a surgeon in validating an implant placement according to some examples as described herein;
- Figure 4: shows a method of DDR rendering according to some examples as described herein;
- Figure 5: shows a screenshot of a comparison of a patient's anatomy before and after the placement of an implant according to some examples as described herein;
- Figure 6: shows a screenshot of a difference between a planned implant placement and an actual implant placement according to some examples as described herein;
- Figure 7: shows a screenshot of a comparison of a patient's anatomy before and after the placement of an implant according to some examples as described herein; and
- Figure 8: shows a block diagram of a system for supporting a surgeon in validating an implant placement according to some examples as described herein.

### Detailed Description

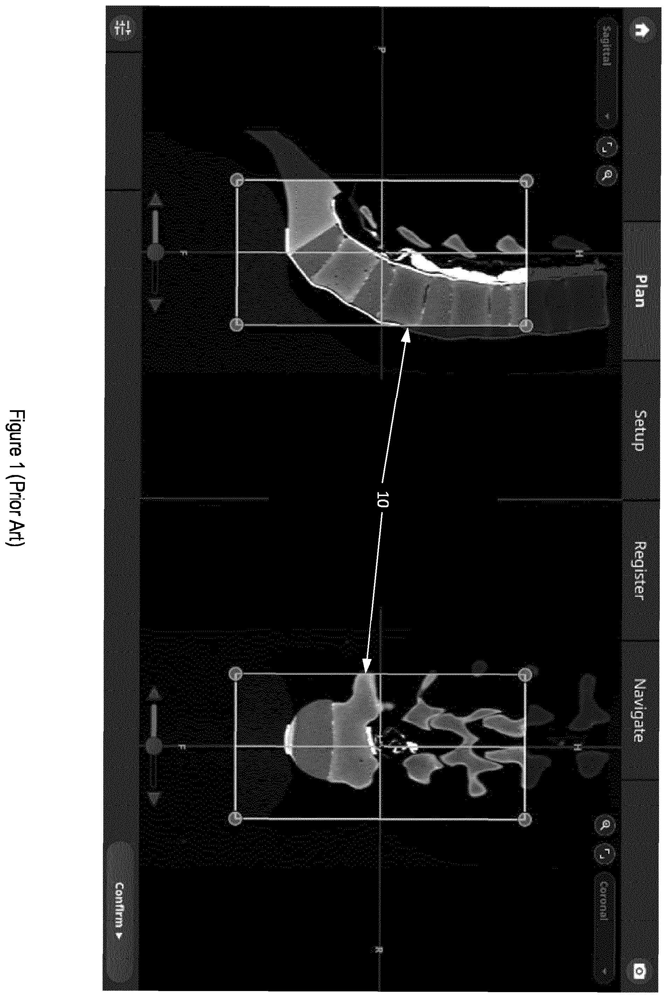

Figure 1 shows a screenshot of a known technique for validating an implant placement. As can be seen, the surgeon can be provided with two DRRs, generated from a post-operative CT scan, with one DRR (in Figure 1, the left image) representing a LAT 2D image showing a plurality of vertebrae 10 and the other an AP 2D image (in Figure 1, the right image) showing the same plurality of vertebrae 10. The surgeon may then check whether the implant as shown in the DRRs is located at the desired position.

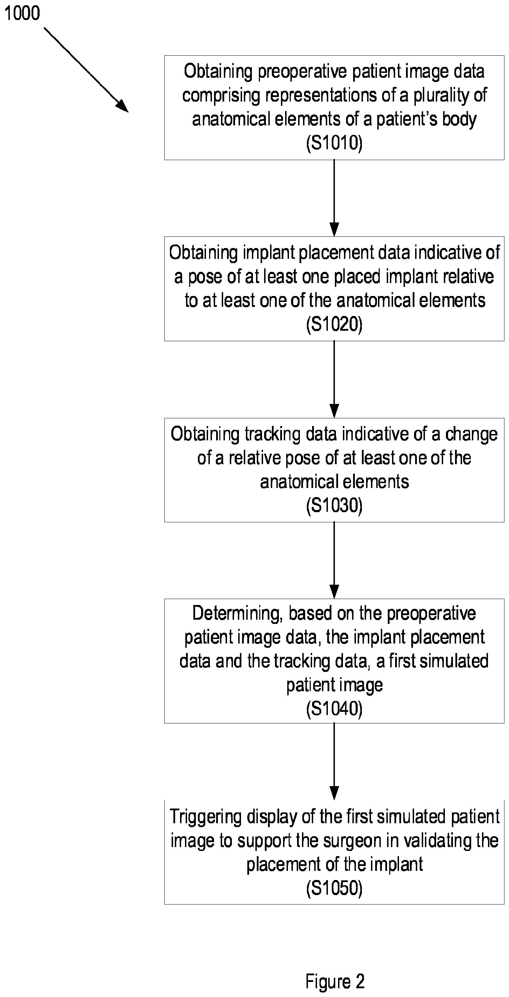

Figure 2 shows a flowchart of supporting a surgeon in validating an implant placement according to some examples as described herein.

The method 1000 described herein is a method 1000 for supporting a surgeon in validating an implant placement. Preferably, this method 1000 is a computer-implemented method, which does not comprise a surgical step. The steps of the method 1000 can be undertaken via, for example, a processor and a memory, or any other suitable means that allows for the method 1000 to take place. The method 1000 may be undertaken entirely on physical hardware, partially on physical hardware and partially in the cloud, or entirely in the cloud, dependent on the scenario the method 1000 is being used in.

The method 1000, in a first step, comprises obtaining S1010 preoperative patient image data comprising representations of a plurality of anatomical elements of a patient's body. The preoperative patient data may be obtained from an external source comprising means for capturing said data. In particular, the external source may be, for example, an X-ray scanner or a CT scanner. The preoperative image data will be described in more detail below.

The method 1000 further comprises obtaining S1020 implant placement data indicative of a pose of at least one placed implant relative to at least one of the anatomical elements. The implant placement data may be predetermined. This data may be based on input data input by the surgeon, or another user of the method 1000. That is to say, this placement data may be input by the surgeon or other medical professional based on the scenario undertaken by the method 1000 and/or the pose of the anatomical elements of the patient's body. Additionally or alternatively, this data may be generated and obtained after the implant has been placed. In such a case, the data may be obtained from an external source such as, for example, an X-ray scanner or a CT scanner.

The method 1000 further comprises obtaining S1030 tracking data indicative of a change of a relative pose of at least one of the anatomical elements. The tracking data may be obtained from a tracking system, for example an optical tracking system that localizes anatomical trackers attached to respective anatomical features of a patient's body (e.g., different bones).

The method 1000 further comprises determining S1040, based on the preoperative patient image data, the implant placement data and the tracking data, a first simulated patient image comprising representations of the plurality of anatomical elements with the at least one of the anatomical elements having the changed pose, the simulated patient image further comprising a visualization of the placed implant. In other words, the first simulated patient image is based on data from the data obtained in the previous steps of the method 1000.

Finally, display of the first simulated patient image to support the surgeon in validating the placement of the implant is triggered S1050. The first simulated patient image may be displayed to the surgeon using any suitable display means such as, for example, a screen, a monitor, or any other means.

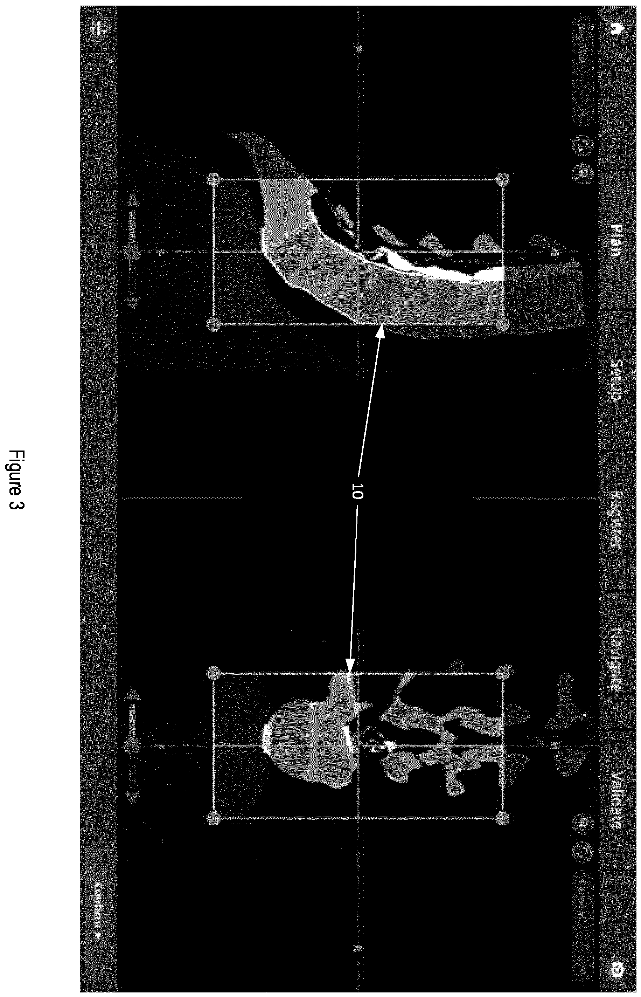

Figure 3 shows a screenshot of supporting a surgeon in validating an implant placement according to some examples as described herein.

Here, similar to Figure 1, the surgeon can be provided with two DRRs, generated from a post-operative CT scan, with one DRR (in Figure 3, the left image) representing a LAT 2D image showing a plurality of vertebrae 10 and the other an AP 2D image (in Figure 3, the right image) showing the same plurality of vertebrae 10. The surgeon may then check whether the implant as shown in the DRRs is located at the desired position.

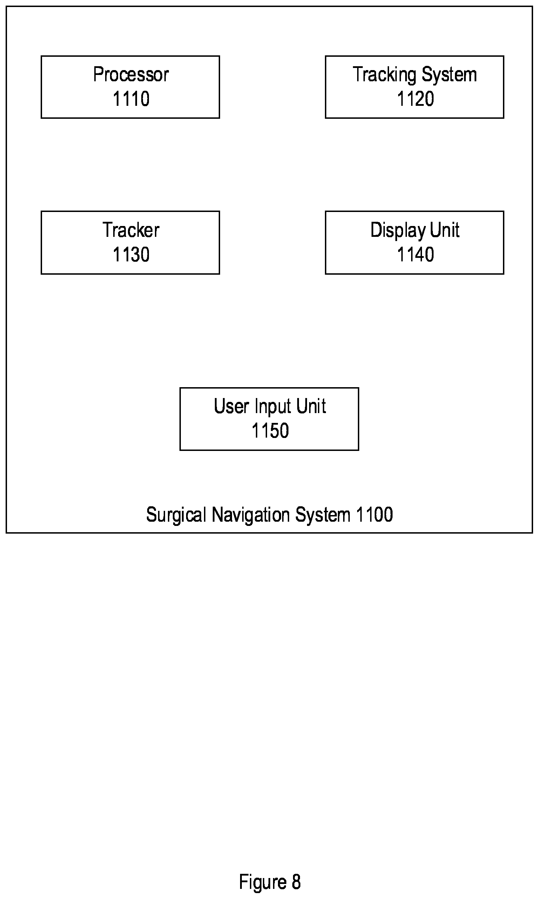

The workflow of the proposed method 1000 may be described in the following. In some examples, not all of the steps mentioned in relation to the method 1000 are required. In some examples, the steps may be in a different order. In some examples, some of the steps happen simultaneously. The method 1000 and/or workflow may be described as follows.
- Import of Navigation Image: A 3D image data set for navigation is imported into the navigation system described herein (See figure 8), for example as part of step S1010.
- Initial Simulated 2D Spine Image: Optionally, an initial Simulated 2D Spine Image is (automatically) generated from the 3D preoperative scan data used for navigation. The image may be generated upon an input by the surgeon, automatically upon presence of a predefined trigger (e.g., upon the implant being inserted, upon a lordotic angle exceeding a predefined threshold, etc.). In some examples, the Initial Simulated Spine Image may be a 3D image.
- Dynamically Changing Patient Model: A patient model may be altered and/or dynamically changed based on:
   a. Rigid structures, known herein as "anatomical elements", such as, for example, vertebrae 10 in the 3D image data set used for navigation are segmented; and/or
   b. During the surgical procedure, anatomical modifications are captured by, for example, tracking multiple rigid structures such as individual vertebrae 10. This may be part of step S1030.
- Capturing Validation Image: Following the completion of a surgical phase, or of the entire procedure, at least one image is incorporated into the proposed Validation section described in further detail below. This may be part of step S1050. The at least one image may correspond to the first simulated patient image.

In some examples, the automatically generated Simulated 2D Spine Images, which may be in, for example, an Anterior - Posterior (AP) and/or Lateral (LAT) View, may be included in and/or incorporated into the capturing of the validation image based on the updated position of the segmented vertebrae 10. The vertebrae 10 may be segmented at any point of the method 1000 before the capturing of the validation image and/or before the generated Simulated 2D Spine Images are included in and/or incorporated into the capturing of the validation image. Segmentation of vertebrae 10 is known to the skilled person.

However, additionally or alternatively, fluoroscopic shots may be imported and included and/or incorporated into the capturing of the validation image and/or 3D scans can be utilized as the foundation for the generating Simulated 2D Spine Images mentioned herein.

A surgical step, for which a validation image is generated, may be an insertion of an implant. The implant, as mentioned and described herein, may be a cage, a plate, a rod, a screw, an artificial disc, an expandable rod, or any other suitable implant. The surgeon may indicate that an implant is inserted by clicking a button, on a display, on a navigated tool such as, for example, a screwdriver, a cage inserter or else. This may trigger the automatic generation of the simulated image.
- Validation workflow step: In the validation section of the navigation system ("Validate" in Figure 3), modifications are visualized for comparison by the surgeon.

Images (imported fluoro shots and/or a Simulated 2D Spine Image) at the end of the surgery or a surgical phase may be displayed in parallel in different windows of the same screen, or on different screens, or are superimposed onto:
a. A pre-operative fluoro shot; and/or
b. The initial generated 2D or 3D spine image, as described above; and/or
c. Any previous intermediate step; and/or
d. An indication of (the placement and/or location of) a planned implant; and/or
e. A second simulated patient image differing from the first simulated patient image.

On the validation page, a history of all images can be visualized and compared in the validation step. In addition, the surgeon can select the images, and image type, used for displaying in the validation step. In particular, the surgeon can choose between 2D or 3D images, should the initial generated spine image be chosen. Additionally or alternatively, the surgeon may choose if the images are displayed in parallel, or are superimposed onto each other. In some examples, at least one of these choices may be predetermined.

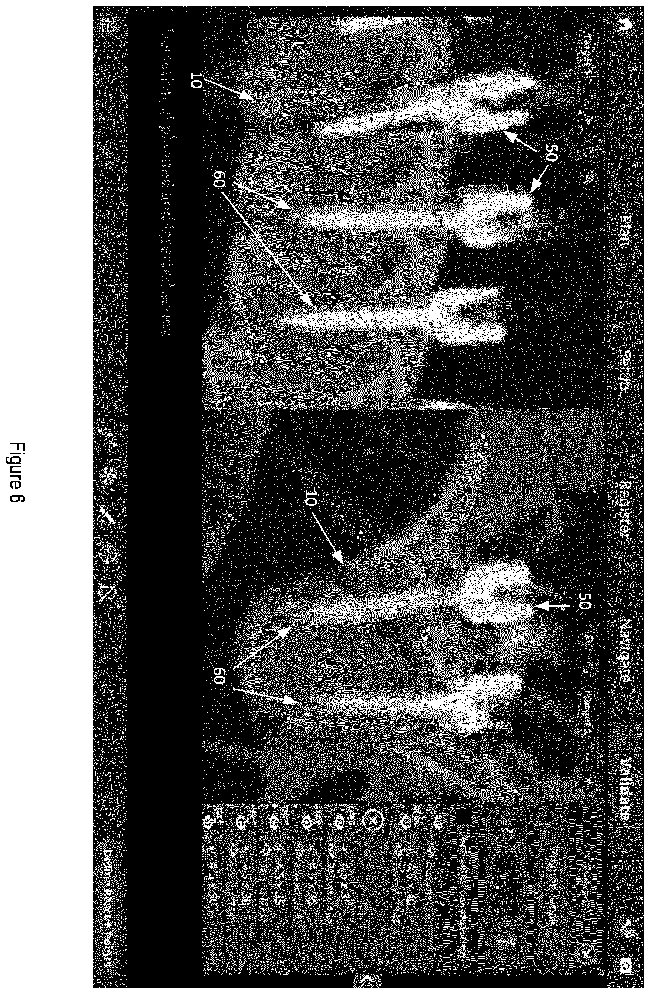

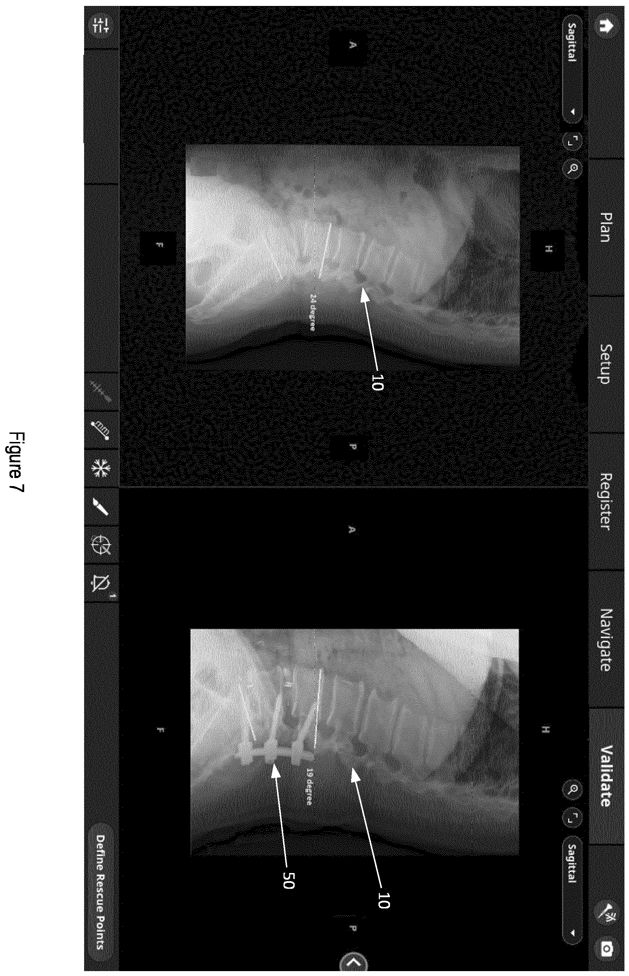

Important statistics such as, for example, various angles such as the lordotic angle and/or a kyphotic angle and/or a Cobb angle between fused vertebrae (see Figure 7) may be displayed. The statistics may comprise, for example, the Gertzbein-Robbins grading scale for screw placement, which is known to the skilled person, and/or placement data indicative of a pose of at least one further placed implant relative to at least one of the vertebrae 10 and/or a deviation between the pose of at least one planned implant and the pose of the at least one placed implant (see Figure 6). Additionally or alternatively, a curve visualizing a planned spinal curvature of the immobilized vertebrae, or a planned rod displayed might be shown for comparison in the validation step.

Simulated 2D Spine Images can be displayed as
- Simulations of C-arm images (Digital Reconstructed Radiographs, DRRs) displayed from different perspectives such as Lateral and/or AP Views. The images can optionally be created with the same perspective as a real fluoro shot for better comparison; or
- Slice reconstructions of 3D image data sets including different perspectives such as, for example, the Axial and/or Coronal and/or Sagittal axes of the vertebra(e) 10 and/or implant related perspectives, such as, for example, slices containing a suitable implant axis. The perspective may be, in some examples, (precisely) aligned to at least one local anatomical structure. This may comprise, for example, showing a Coronal view of a vertebra 10 including a "symmetrical" view of the spinous process and both transverse processes.

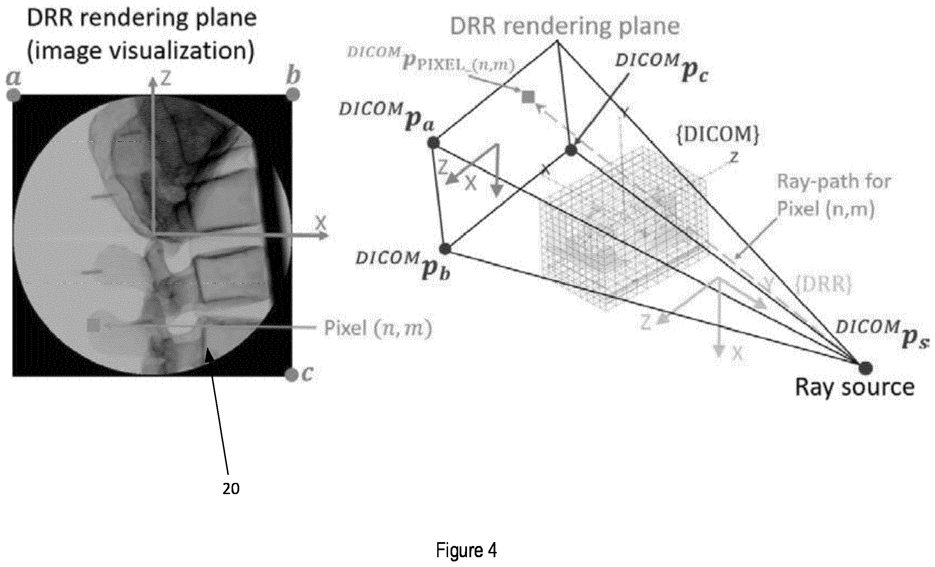

Figure 4 shows a method of DDR rendering according to some examples as described herein. This method may be used to generate the first simulated patient image.

In a first step, the navigation image dataset undergoes updating based on the tracked vertebrae 20. This involves capturing the position by attaching trackers to at least one vertebra, and subsequently updating a model containing the segmented vertebra 20, or vertebrae 20 in the case of multiple vertebrae being tracked. Data relating to the positioning of the tracker may be used, in some examples, as the tracking data, to generate the first simulated patient image as described herein. In some examples, implant placement data is determined based on tracking data, which may be received and/or based on the tracker, indicative of a pose of the at least one placed implant and tracking data, which may be received and/or based on the tracker, indicative of a simultaneous pose of the at least one of the anatomical elements. In some examples, only a subset of the vertebrae 20 are segmented. These tracked vertebrae 20 may be the same as the vertebrae 10 mentioned in relation to Figures 3, 5 and 6, or may be other vertebrae.

Following this, a (e.g., state-of-the-art) deformable registration method is employed. Here, only the bony structures (e.g., the vertebrae) are treated as rigid during the registration process.

Optionally, tools for bone resectioning can be tracked by capturing the trajectory of a tool tip while it traverses bony structures. This facilitates the measurement of the volume of resected bone, which can subsequently be subtracted from the original 3D image dataset (the preoperative patient image data) prior to generation of the Simulated 2D Spine Image (e.g., the first simulated patient image). The image after the deformable registration has been applied can be directly used to create 2D slices for visualization (slice reconstruction).

Another option is to create a simulated 2D fluoro shot, which may be associated with the DRR method disclosed herein. An algorithm for generating said DDR is the Seddon and Jacobs algorithm, which creates digitally reconstructed radiographs out of 3D image data sets, although any other suitable algorithm may additionally or alternatively be used. The Seddon and Jacobs algorithm is a ray casting algorithm with the ray source at the isocenter of a simulated C-Arm. All Hounsfield unit values on a single ray are integrated (summed up) to render a pixel on the DRR. The process is repeated for each of the pixel of the DRR. Figure 4 depicts the DRR rendering process for the pixels in the simulated 2D shot.

The 2D shot may be simulated as per the selected anatomical plane such as, for example, the coronal and/or sagittal and/or axial plane. The perspective may be finetuned by aligning the 2D shot with a previously captured real fluoro shot. Furthermore, an axis and/or a linear border of the vertebra(e) 20, as represented in the first simulated patient image, may be triggered to be displayed in response to obtained user input, wherein the surgeon selects the axis and/or linear border to be displayed.

To visualize bony structures and/or soft tissues, the Hounsfield unit values can be filtered. It may be possible to differentiate between soft tissues and bony structures based on the Hounsfield value ranges. Soft tissues typically have Hounsfield unit values ranging from about 0 to about 100 HU on a typical CT scan. However, the exact Hounsfield unit values can vary depending on factors such as, for example, the specific soft tissue being imaged. Bones typically have higher Hounsfield unit, HU, values compared to soft tissues due to their greater density. The exact HU values for bones can vary depending on factors such as, for example, the type of bone and its composition. However, the HU values for bones typically range from about 700 to about 3000 HU.

The user is preferably provided with an option to render/filter out the soft tissues by selecting appropriate ranges of Hounsfield unit values. That is to say, the surgeon may be given the option to eliminate the soft tissue in the generated image by electing to show only tissues/elements with a HU value greater than 500, or any other suitable value, in the generated image. Additionally or alternatively, this filtering may be preprogrammed into the method 1000 and/or automatically determined as part of the method 1000.

To include implants, such as screws, in the generated image, the implant attributes such as, for example, position and/or geometry and/or material may be considered. The respective volume of the implant in the image data set may be calculated. An object with the appropriate HU values matching the implant material is placed in the 3D image data set. The same can be applied to other implants such as, for example, cages. After placing the object, the DRR is computed.

For rendering a Slice Reconstruction, the mesh of the planned implant may be added to the 3D data set. An appropriate plane is chosen by the surgeon and/or the plane is predetermined and/or the plane is preprogrammed into the method 1000 described herein. Planes which are preferably used may include the implant trajectory such as the Implant Axial plane (Target 2 in Figure 6) and the Implant Sagittal plane (Target 1 in Figure 6). The mesh of the implant is intersected with the selected plane, and the resulting contour is visualized in the image displayed to the surgeon (see Figure 6) in the first simulated patient image.

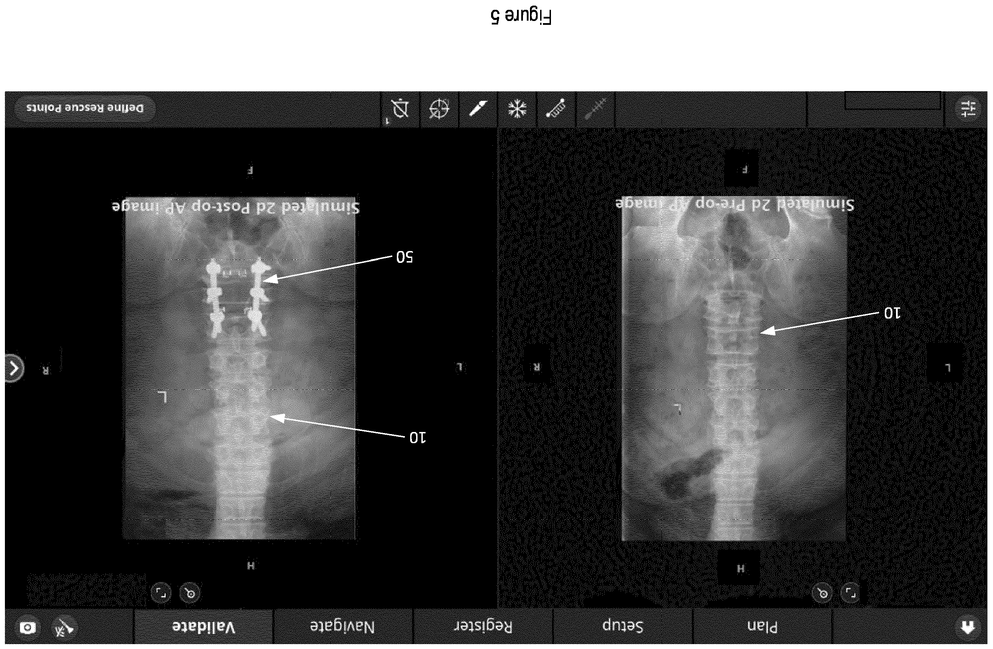

Figure 5 shows an exemplary visualization that may be displayed based on step S1050. This visualization provides a comparison of a patient's anatomy before and after the placement of an implant 50 according to some examples as described herein. As can be seen, the images are similar in terms of the view and perspective of the vertebrae 10 within the images, but differ in that in the preoperative Simulated 2D Spine Image (left in Figure 5), no implants 50 are visible, whereas in an adjacently displayed intra- or post-operative Simulated 2D Spine Image (right in Figure 5), implants 50 are visualized as they would appear in an x-ray image.

Figure 6 shows an exemplary visualization that may be displayed based on step S1050. This visualization not only shows the intra- or postoperative Simulated 2D Spine Image comprising the vertebrae 10, but also indicates planned poses 60 of the implants by overlaying outlines of the planned implants 60 on top of the actually placed implants 50. This helps in identifying a difference between a planned implant placement 50 and an actual implant placement 50 according to some examples as described herein.

Figure 7 shows an exemplary visualization that may be displayed based on step S1050. This visualization provides a comparison of a patient's anatomy before and after the placement of an implant 50 according to some examples as described herein. As can be seen, the images are similar in terms of the view and perspective of the vertebrae 10 within the images, but differ in that in the preoperative Simulated 2D Spine Image (left in Figure 5), no implants are visible, whereas in an adjacently displayed intra- or post-operative Simulated 2D Spine Image (right in Figure 5), implants 50 are visualized as they would appear in an x-ray image. The visualization also indicates the poses of endplates of two vertebrae 10. This helps the surgeon in comparing the spinal alignment before and after implant 50 placement.

The Validation workflow screen as exemplarily shown in Figs. 5, 6 and 7, may be triggered at any time by clicking on the Validation/Validate tab (see Figure 3). In particular it may be triggered:
1. After changing the anatomy of the patient by, for example, performing disc spreading, bone resection; and/or
2. After placement of an implant 50; and/or
3. Once all the implants 50 are placed and procedure is finished.

When entering the validation workflow by clicking the Validation/Validate tab, the anatomical area where the last implant 50 was inserted may be automatically identified (e.g., based on tracking the implant placement instrument such as screwdriver). The region may be zoomed in automatically. A comparison between the last displayed anatomical state and the validation image may be shown as default view. Additionally or alternatively, the surgeon can compare the complete spine structure, or select a customized area for comparison. In some examples, the surgeon may pick a previous state/image from the image history. In some examples, the surgeon can select the display mode (slice reconstruction or DRR) and the perspective, such as, for example, the Axial and/or Sagittal and/or Coronal axis, which is aligned to a specific fluoro shot, which itself is aligned to a specific implant 50.

The Validation tab may offer the option to generate a report. Upon selecting this option, a final report is generated including a comprehensive comparison between the original and modified spine structures. That is to say, a comparison between the preoperative patient image data and the first simulated patient image. Additionally, it may, in some examples, feature comparisons between the original spine and modified spine (after the implant 50 has been placed) at the location of each implant 50.

In some examples, the first simulated patent image may be generated for all images which are not directly imported 3D or 2D image datasets to inform the surgeon that the images are estimations or approximations of patient data acquired by imaging devices.

For workflow documentation, generated images may be generated and/or included after each placed implant (e.g., the AP and/or lateral views). A time stamp may be added for each generated image. A timeline may additionally or alternatively be included. This timeline may show a chronological series of generated images. In cases where implants 50 are pre-planned, documenting deviations between the planned 60 and placed implant 50 locations and/or positions (e.g., screw tip, screw head, and transverse angle) can enhance the clarity of the record. Additionally, considering the documentation of the lordotic/kyphotic/Cobb angle between the fused vertebrae 10 (see Figure 7) before and after surgery may provide valuable insight. Such a difference may, in some examples, be displayed to the surgeon when the first simulated patient image is triggered to be displayed to the surgeon. Furthermore, such a difference may be based on the vertebrae 20 being tracked with, or without, the use of a tracker mentioned herein. Documenting the chosen implant 50 family and size for comprehensive record-keeping may also be part of the presently described method 1000. Optionally, highlighting resected bony structures may offer additional context to the surgery. For each screw the Gertzbein-Robbins grading scale, as mentioned above and known to the skilled person, may be computed and/or displayed.

Figure 8 shows a block diagram of a system for supporting a surgeon in validating an implant placement according to some examples as described herein.

The Surgical Navigation System 1100 may employ the method 1000 described herein. said System 1100 comprises a processor 1110 and, when required, at least one of a Tracking System 1120, a Tracker 1130, a Display Unit 1140 and a User Input Unit 1150. Said elements 1120, 1130, 1140, 1150 may use any suitable process known to the skilled person in order to undertake the method 1000 as described herein.

Generally speaking, the following challenges may be present for supporting a surgeon in the field of implant placements:
• Patient and surgeon exposure to X-rays for 2D shots at the end of the surgery and after completion of various surgical procedures such as placing a pedicle screw;
• Manual review of 2D shot or comparison of pre- and post-surgery shot is required;
• No statistics or parameter comparison;
• No validation step at the end of the workflow; and
• Manual documentation of the surgical procedure.

The technique as described herein may have at least the following advantages:
• Less exposure to radiation, if the surgeon uses the navigation system to verify results rather than capturing a 2D shot;
• A validation step in the workflow;
• Better evaluation and comparison of results with all statistics;
• Quicker, more convenient, and time saving for the validation process; and
• Can be used for generating a final patient report and associated documentation.

No doubt many other effective alternatives will occur to the skilled person. It will be understood that the invention is not limited to the described embodiments and encompasses modifications apparent to those skilled in the art and lying within the scope of the claims appended hereto.

## Claims

1. A computer-implemented method (1000) for supporting a surgeon in validating an implant placement, the method (1000) comprising:
obtaining (S1010) preoperative patient image data comprising representations of a plurality of anatomical elements of a patient's body;
obtaining (S1020) implant placement data indicative of a pose of at least one placed implant relative to at least one of the anatomical elements;
obtaining (S1030) tracking data indicative of a change of a relative pose of at least one of the anatomical elements;
determining (S1040), based on the preoperative patient image data, the implant placement data and the tracking data, a first simulated patient image comprising representations of the plurality of anatomical elements with the at least one of the anatomical elements having the changed pose, the simulated patient image further comprising a visualization of the placed implant; and
triggering (S1050) display of the first simulated patient image to support the surgeon in validating the placement of the implant.

2. The method (1000) of claim 1, wherein the preoperative patient image data comprises 3D image data and the first simulated patient image is a 2D image or a 3D image.

3. The method (1000) of claim 1 or 2, wherein the tracking data is obtained (S1030) via at least one tracker attached to at least one of the anatomical elements, and wherein the first simulated patient image is determined (S1040) at least partly based on the tracking data obtained (S1030) via the at least one tracker.

4. The method (1000) of any one of the preceding claims, wherein the first simulated patient image is determined (S1040) at least partly based on a computer-implemented deformable registration method, wherein an input of the computer-implemented deformable registration method is the obtained (S1030) tracking data.

5. The method (1000) of any one of the preceding claims, wherein the implant placement data is determined based on tracking data indicative of a pose of the at least one placed implant and tracking data indicative of a simultaneous pose of the at least one of the anatomical elements.

6. The method (1000) of any one of the preceding claims, further comprising:
adding a mesh of one or more of the at least one implant to the preoperative patient image data; and
intersecting the mesh with an image plane of the first simulate patient image that is to be determined,
wherein an indication of an implant contour resulting from the intersecting is visualized in the first simulated patient image.

7. The method (1000) of any one of the preceding claims, wherein the first simulated patient image is constructed with respect to a plane parallel with a longitudinal axis of one or more of the at least one placed implant, or a plane perpendicular to the longitudinal axis of one or more of the at least one placed implant.

8. The method (1000) of any one of the preceding claims, wherein the first simulated patient image is determined (S1040) at least partly based on a computer-implemented ray casting algorithm configured to generate a Hounsfield unit value for at least part of the at least one of the anatomical elements, and wherein the generated Hounsfield unit value is configured to be used to generate a Digitally Reconstructed Radiograph, DRR, for the first simulated patient image.

9. The method (1000) of claim 8, further comprising, before or after triggering (S1050) the display of the first simulated patient image, altering the first simulated patient image based on the generated Hounsfield unit value, in particular wherein the first simulated patient image is altered based on one or more of:
a first Hounsfield unit value range corresponding to soft tissue;
a second Hounsfield unit value range corresponding to bone; and
a third Hounsfield unit value range corresponding to the at least one placed implant.

10. The method (1000) of claim 8 or 9, wherein the implant placement data further comprises a volume of the at least one placed implant, and wherein a virtual object with a Hounsfield unit value matching a material of the implant is placed into the preoperative patient image data.

11. The method (1000) of any one of the preceding claims, wherein the simulated first patient image is triggered (S1050) to be displayed together with at least one of:
- a pre-operative medical patient image such as a fluoroscopic image;
- an initial simulated patient image;
- an indication of a result of an intermediate step of the method;
- an indication of a planned implant;
- a second simulated patient image differing from the first simulated patient image.

12. The method (1000) of any one of the preceding claims, further comprising:
determining a difference between a pose of at least one planned implant and a pose of the at least one placed implant, and triggering display of an indication of the difference.

13. The method (1000) of any one of the preceding claims, further comprising:
determining a deviation between a pose of an anatomical element as represented by the patient image and a changed pose of the anatomical element as indicated by the tracking data, and triggering display of an indication of the deviation.

14. The method (1000) of any one of the preceding claims, further comprising obtaining user input, wherein the first simulated patient image is triggered to be displayed in response to the obtained user input.

15. The method of (1000) claim 14, wherein an axis and/or a linear border of the at least one of the anatomical elements as represented in the first simulated patient image is triggered to be displayed in response to the obtained user input.

16. The method (1000) of any one of the preceding claims, wherein an indication of at least one spinal parameter derived from the first simulated patient image is triggered to be displayed together with the first simulated patient image.

17. The method (1000) of claim 16, wherein the at least one spinal parameter comprises one or more of:
- an indication of a Gertzbein-Robbins grading scale;
- a lordotic angle and/or a kyphotic angle and/or a Cobb angle between different ones of the plurality of anatomical elements, in particular between fused vertebrae;
- a curve visualizing a curvature of the plurality of anatomical elements, in particular a spinal curvature of immobilized vertebrae;
- placement data indicative of a pose of at least one further placed implant relative to at least one of the anatomical elements, in particular a rod; and
- a deviation between the pose of at least one planned implant and the pose of the at least one placed implant, in particular wherein the implant is a screw.

18. A surgical navigation system (1100) comprising at least one processor (1110) configured to carry out the method (1000) of any one of the preceding claims.

19. The surgical navigation system (1100) of claim 18, further comprising at least one of the following entities:
a tracking system (1120) configured to generate the tracking data;
at least one tracker (1130) trackable by the tracking system and attachable to at least one anatomical element of the patient;
a display unit (1140) configured to display at least the first simulated patient image;
a user input unit (1150) configured to receive user input.

20. A computer program comprising instructions which, when the program is executed by at least one processor, cause the at least one processor to carry out the method (1000) of any one of claims 1 to 19, wherein the program is optionally carried by a carrier such as a data signal, a portable memory device or a non-transitory computer storage medium.

## Amended claims

### Amended claims in accordance with Rule 137(2) EPC.

1. A computer-implemented method (1000) for supporting a surgeon in validating an implant placement which does not comprise a surgical step, the method (1000) comprising:
obtaining (S1010) preoperative patient image data comprising representations of a plurality of anatomical elements of a patient's body;
obtaining (S1020) implant placement data indicative of a pose of at least one placed implant relative to at least one of the anatomical elements;
obtaining (S1030) tracking data indicative of a change of a relative pose of at least one of the anatomical elements;
determining (S1040), based on the preoperative patient image data, the implant placement data and the tracking data, a first simulated patient image comprising representations of the plurality of anatomical elements with the at least one of the anatomical elements having the changed pose, the simulated patient image further comprising a visualization of the placed implant; and
triggering (S1050) display of the first simulated patient image to support the surgeon in validating the placement of the implant.

2. The method (1000) of claim 1, wherein the preoperative patient image data comprises 3D image data and the first simulated patient image is a 2D image or a 3D image.

3. The method (1000) of claim 1 or 2, wherein the tracking data is obtained (S1030) via at least one tracker attached to at least one of the anatomical elements, and wherein the first simulated patient image is determined (S1040) at least partly based on the tracking data obtained (S1030) via the at least one tracker.

4. The method (1000) of any one of the preceding claims, wherein the first simulated patient image is determined (S1040) at least partly based on a computer-implemented deformable registration method, wherein an input of the computer-implemented deformable registration method is the obtained (S1030) tracking data.

5. The method (1000) of any one of the preceding claims, wherein the implant placement data is determined based on tracking data indicative of a pose of the at least one placed implant and tracking data indicative of a simultaneous pose of the at least one of the anatomical elements.

6. The method (1000) of any one of the preceding claims, further comprising:
adding a mesh of one or more of the at least one implant to the preoperative patient image data; and
intersecting the mesh with an image plane of the first simulated patient image that is to be determined,
wherein an indication of an implant contour resulting from the intersecting is visualized in the first simulated patient image.

7. The method (1000) of any one of the preceding claims, wherein the first simulated patient image is constructed with respect to a plane parallel with a longitudinal axis of one or more of the at least one placed implant, or a plane perpendicular to the longitudinal axis of one or more of the at least one placed implant.

8. The method (1000) of any one of the preceding claims, wherein the first simulated patient image is determined (S1040) at least partly based on a computer-implemented ray casting algorithm configured to generate a Hounsfield unit value for at least part of the at least one of the anatomical elements, and wherein the generated Hounsfield unit value is configured to be used to generate a Digitally Reconstructed Radiograph, DRR, for the first simulated patient image.

9. The method (1000) of claim 8, further comprising, before or after triggering (S1050) the display of the first simulated patient image, altering the first simulated patient image based on the generated Hounsfield unit value, in particular wherein the first simulated patient image is altered based on one or more of:
a first Hounsfield unit value range corresponding to soft tissue;
a second Hounsfield unit value range corresponding to bone; and
a third Hounsfield unit value range corresponding to the at least one placed implant.

10. The method (1000) of claim 8 or 9, wherein the implant placement data further comprises a volume of the at least one placed implant, and wherein a virtual object with a Hounsfield unit value matching a material of the implant is placed into the preoperative patient image data.

11. The method (1000) of any one of the preceding claims, wherein the simulated first patient image is triggered (S1050) to be displayed together with at least one of:
- a pre-operative medical patient image such as a fluoroscopic image;
- an initial simulated patient image;
- an indication of a result of an intermediate step of the method;
- an indication of a planned implant;
- a second simulated patient image differing from the first simulated patient image.

12. The method (1000) of any one of the preceding claims, further comprising:
determining a difference between a pose of at least one planned implant and a pose of the at least one placed implant, and triggering display of an indication of the difference.

13. The method (1000) of any one of the preceding claims, further comprising:
determining a deviation between a pose of an anatomical element as represented by the patient image and a changed pose of the anatomical element as indicated by the tracking data, and triggering display of an indication of the deviation.

14. The method (1000) of any one of the preceding claims, further comprising obtaining user input, wherein the first simulated patient image is triggered to be displayed in response to the obtained user input.

15. The method of (1000) claim 14, wherein an axis and/or a linear border of the at least one of the anatomical elements as represented in the first simulated patient image is triggered to be displayed in response to the obtained user input.

16. The method (1000) of any one of the preceding claims, wherein an indication of at least one spinal parameter derived from the first simulated patient image is triggered to be displayed together with the first simulated patient image.

17. The method (1000) of claim 16, wherein the at least one spinal parameter comprises one or more of:
- an indication of a Gertzbein-Robbins grading scale;
- a lordotic angle and/or a kyphotic angle and/or a Cobb angle between different ones of the plurality of anatomical elements, in particular between fused vertebrae;
- a curve visualizing a curvature of the plurality of anatomical elements, in particular a spinal curvature of immobilized vertebrae;
- placement data indicative of a pose of at least one further placed implant relative to at least one of the anatomical elements, in particular a rod; and
- a deviation between the pose of at least one planned implant and the pose of the at least one placed implant, in particular wherein the implant is a screw.

18. A surgical navigation system (1100) comprising at least one processor (1110) configured to carry out the method (1000) of any one of the preceding claims.

19. The surgical navigation system (1100) of claim 18, further comprising at least one of the following entities:
a tracking system (1120) configured to generate the tracking data;
at least one tracker (1130) trackable by the tracking system and attachable to at least one anatomical element of the patient;
a display unit (1140) configured to display at least the first simulated patient image;
a user input unit (1150) configured to receive user input.

20. A computer program comprising instructions which, when the program is executed by at least one processor, cause the at least one processor to carry out the method (1000) of any one of claims 1 to 17, wherein the program is optionally carried by a carrier such as a data signal, a portable memory device or a non-transitory computer storage medium.
